# EUROPEAN PATENT SPECIFICATION

(11) **EP 3 769 610 B1**
(45) Date of publication and mention of the grant of the patent: **07.06.2023**
(21) Application number: 19772411.5
(22) Date of filing: 22.01.2019
(51) Int. Cl.: A01G 18/20, A01G 18/66, A01G 18/70

(54) **PLANTING METHOD FOR MORELS**
PFLANZVERFAHREN FÜR MORCHELN
PROCÉDÉ DE PLANTATION DE MORILLES

(30) Priority: 21.03.2018 CN 201810235910
(43) Date of publication of application: 27.01.2021
(73) Proprietor: Sichuan Three Point Water Biotechnology Co., Ltd., Mianyang, Sichuan 621000 (CN)
(72) Inventor: XIE, Linsen, Mianyang, Sichuan 621000 (CN)
(74) Representative: Murgitroyd & Company
(86) International application number: PCT/CN2019/072636
(87) International publication number: WO 2019/179231

(56) References cited:
- WO-A2-00/23331
- CN-A- 104 956 922
- CN-A- 105 993 590
- CN-A- 106 358 751
- CN-A- 106 358 751
- CN-A- 106 576 901
- CN-A- 107 439 221
- CN-A- 107 567 954
- CN-A- 107 567 954
- LIU QIZHENG ET AL: "Artificial cultivation of true morels: current state, issues and perspectives", CRITICAL REVIEWS IN BIOTECHNOLOGY, vol. 38, no. 2, 6 June 2017 (2017-06-06), pages 259-271, XP055790851, US ISSN: 0738-8551, DOI: 10.1080/07388551.2017.1333082 Retrieved from the Internet: URL:https://www.tandfonline.com/doi/pdf/10 .1080/07388551.2017.1333082?needAccess=tru e>

## Description

### TECHNICAL FIELD

The disclosure relates to a planting method for morels.

### BACKGROUND

Morel is a rare edible fungus, named after a rugged and goat-tripe-like pileus thereof. Morel, also known as Morchella mushroom, sponge mushroom, or pinecone mushroom, is a rare edible and medicinal fungus for treating indigestion, qi stagnation, abdominal fullness and distention, and dyspnea with cough caused by accumulation of phlegm and qi counterflow.

The morel is the best-known delicious edible fungus among Ascomycetes, the pileus thereof contains seven essential amino acids to human body: isoleucine, leucine, lysine, methionine, phenylalanine, threonine, and valine. The morel is sweet in taste, cold in nature, and nontoxic. The fungus benefits intestines and stomach and regulates qi -flowing for eliminating phlegm.

Morels are fairly nutritious. It has been determined that a morel contains crude protein (20%), crude fat (26%), carbohydrates (38.1%), and a plurality of amino acids, and particularly glutamate content is as high as 1.76%. Therefore, morel is believed as "an extremely good protein source" and has a reputation of "meat of vegetarian diet". In the human body, proteins are composed of 20 amino acids; the morel includes 18 ones, eight of which cannot be produced in the human body but are especially important in human nutrition, known as "essential amino acids". In addition, it has been determined that there are at least eight vitamins in the morel: vitamins B1, B2 and B12, nicotinic acid, pantothenic acid, pyridoxine, biotin, folic acid, etc. The morel has nutrients comparable to milk, meat and fish meal. Therefore, the morel is often called one of "healthy foods" internationally. The morel contains tumor suppressor polysaccharides and antimicrobial and antiviral active components, which has immunopotentiating, antifatigue, antiviral, and tumor suppressor effects; Japanese scientists have found that morel extract contains a tyrosinase inhibitor that effectively inhibits the formation of lipofuscin. Rich selenium in the morel is a component of glutathione peroxidase in human red blood cell, which can transport a number of oxygen molecules to inhibit malignant tumors and inactivate cancer cells; on the other hand, antioxidation of vitamin E can be enhanced. The antioxidation of selenium can change the metabolic direction of carcinogens, and selenium binds to carcinogens for detoxification, thereby reducing or eliminating the risk of cancers.

The existing planting method for morels can be summarized in the following steps: preparing strains, seeding strains, covering soil, preparing a mushroom strain bag, mycelia coming up, placing the mushroom strain bag, managing the humidity, removing the mushroom strain bag, and fruiting. Specifically, well-mixed composts are bagged and sterilized under normal or high pressure, and then a strain is inoculated in an aseptic environment; after dozens of days, mycelia are full of the bag, and then the bag is placed for 5-10 days to allow mycelia to penetrate the composts; well-cultured strains are triturated and broadcast-strained evenly in the field, and then 2-3 cm of soil is covered; after that, a perforated film is mulched optionally; the mushroom strain bag is sterilized under normal or high pressure until mycelia grow all over the soil surface and appear white; after cooling and breaking, the mushroom strain bag is placed on the ground; after a month, the mushroom strain bag is removed until overgrowth; after managing the humidity, morels can fruit for harvesting.

The above planting method has the following defects:
- 1. The yield is unstable; the yield ranges from 0 to 800 catties, with an average yield of around 200 catties.
- 2. There are a number of planting steps and planting essentials, and these planting essentials are not easy to master; it is difficult to grasp the timing of placement of the mushroom strain bag, i.e., early placement readily leads to undesired microbial infection, and late placement leads to low yield, and even no yield.

CN 107 567 954 (TAIHU COUNTY JINJIANGYUAN AGRICULTURAL DEV CO LTD) relates to a morchella cultivation method which includes the steps: base material culture; sterilization; inoculation; hyphae culture; land treatment; awning building; secondary plowing; field deed breeding; final-period management. By the aid of common raw materials, grain raw materials are avoided or decreased, grain resources are saved, cost is reduced, a lot of straws are consumed, waste is turned into wealth, air pollution caused by straw burning is avoided, parts of applied strains not consumed by morchella further can serve as organic fertilizers, soil is improved, and nutriment can be provided for crops of a next season.

WO0023331 (HSU WEI K) relates to a sterilizable cultivation system provided for growing selected organisms for use in biological applications and the cultivation edible fungi. A sealable, sterilizable container holds a growth substrate in a sterile condition. A separate, adhesive vent filter is provided, which is adhereable to a vent opening in the container. The vent filter is gas permeable and allows gas exchange between the interior of the container and the ambient environment, but prevents the passage of microbes and other organisms into or out of the container. This gas exchange prevents excess carbon dioxide from accumulating in the container and allows necessary oxygen into the container in order to maintain an appropriate growth environment for the selected organisms. The vent filter may be combined with the container (a) during manufacture of new containers, or (b) in the field by the end-user on new containers or on re-cycled containers. The separately provided vent filters allow the end-user to adjust certain parameters of the growth environment within the container in the field. This is accomplished by adjusting the venting characteristics of the cultivation system, by increasing or reducing the amount and quality of the vent filters on the container.

Artificial cultivation of true morels: current state, issues and perspectives (ISSN 0738-8551) (Liu Qizheng ET AL) relates to the life cycle and reproductive systems in the genus Morchella and the current state of outdoor cultivation.

CN106358751 (SOIL & FERTILIZER RES INST SAAS) relates to edible mushroom cultivation, in particular relates to a method for cultivating morchella, and aims to solve the technical problem of providing a novel method for cultivating morchella. The main improvement is that the method is achieved by adopting a cultivation mode and a novel cultivation medium in match. The method provided by the invention comprises the following steps: A, pretreating a cultivation land; B, opening ridges, and putting morchella bags on the ground; C, covering the morchella bags with soil, and constructing the ridges; D, growing the morchella; E, placing nutrient bags; and F, inducing the morchella till the morchella is harvested. By adopting the method provided by the invention, the cost of each mu of the land can be reduced, moreover, the yield of the morchella can be remarkably increased, and the method is worthy of popularization and application.

CN105993590 (UNIV SOUTHWEST SCIENCE & TECH) relates to a culturing method for sporocarp of Morchella. The culturing method comprises the following steps: fungal strain preparation, fungal strain culturing, soil treatment, sowing, covering and temperature preservation, management in the growth period of mycelia, fruiting acceleration treatment, management in a fruiting period, and harvesting. According to the invention, operation steps like drawing of fungal strains from a fungal strain bottle or a fungal strain bag, fungal strain hydration, and placement of a large amount of nutrition material bags on the surface of soil in a traditional technology are removed, so the field-cultivation or indoor-cultivation technique for the Morchella is more simple and convenient.

### SUMMARY

To overcome defects that planting methods for morels have a number of steps and are difficult to grasp, with low and unstable yield, the disclosure provides a planting method for morels; the planting method is simple, is easy to learn and promote, has a stable and high yield, and requires few strains, thereby reducing planting costs.

To solve the above technical problems, the disclosure adopts the following technical solutions:
A planting method for morels is provided, including the following steps:
- step 1, preparing spawn, comprising
- 1.1 filling composts (5) and the soil layer an entire mushroom strain bag wherein the composts are compressed and the soil layer is 1-2 cm thick, covering a layer of loose soil (6) on the composts, sealing the mushroom strain bag for sterilization and sterilizing the mushrooms strain bag;
- 1.2 after sterilization, opening the mushroom strain bag for inoculation, inoculating the mushroom strain bag, the mushroom strain bag is sealed and going to the next step until morel mycelia (7) grow down to at least 2-3 cm of the soil layer;
- step 2, flipping the spawn, comprising:
- 2.1 cutting off the mushroom strain bag, flipping on the ground directly, and compressing;
- step 3, managing the humidity, wherein surface soil moisture is required at 70-85%;
- step 4, fruiting for harvesting.

In step 1.1, the soil layer is 1-2 cm thick.

In step 1.1, the composts are available from either those in the existing cultivation of morels, or those prepared by the following method: wheats are soaked in saturated limewater for one day, and then well-soaked corncobs and other components are mixed therein to prepare the composts.

In step 1.1, the mushroom strain bag includes a body and a cap (an air-permeable bottle cap or a bottle cap for edible fungi), the cap is capped on the body, air holes are arranged at the side or bottom of the body, and a layer of air-permeable filter material is covered on the air holes. The mushroom strain bag may also be made from a bag.

In step 1.1, the composts and the soil layer are needed to fill the entire mushroom strain bag, and the composts are needed to be compressed.

In step 1.2, when inoculating, it is determined whether the mushroom strain bag is inoculated in an aseptic environment according to the compactness thereof; after inoculation, the mushroom strain bag is sealed. (If compact, the bag can be inoculated in the presence of microbes, because undesired microbes are difficult to penetrate the soil; if loose, the bag should be inoculated in an aseptic environment.)

In step 2, the spacing between mushroom strain bags is 30-35 cm, surface soil is 16-25 cm thick, and a soil tank is 100-120 cm wide.

In step 2, the mushroom strain bag is flipped on the ground and compressed, and mycelia grow up in a circular manner around the mushroom strain bag.

In step 4, fruiting for harvesting is achieved 70-100 days after the mushroom strain bag is flipped on the ground.

Such conditions as planting temperature and air humidity are subject to the existing planting requirements for morels.

Compared with the prior art, the disclosure has the following beneficial effects:
- 1. The planting method of the disclosure merely includes steps of preparing spawns, flipping the spawns, managing the humidity, and fruiting for harvesting. Planting steps of morels are substantially simplified, and mushroom strain bags are flipped on the ground directly after inoculating the strains. Because a soil layer is covered on the composts, and the soil functions as a barrier, morel mycelia will penetrate the soil into the composts directly, but other undesired fungi do not, so that undesired fungi do not contaminate easily and the yield will increase substantially. Unlike the existing planting methods, the disclosure does not need to take the good chance of the mushroom strain bag, is easy to operate and grasp, and does not influence the yield stability due to operations by different people. Therefore, through the method provided by the disclosure, the yield is not only stable, but also increases substantially, which can reach at least 800 catties per mu. In the disclosure, it is important to cover a layer of soil on the composts, which can keep microbes out well; flipping the mushroom strain bag on the ground is convenient for seeding; after inoculation, the strains are not flipped until mycelia grow down to at least 2-3 cm of the soil layer, so as to fully ensure the inoculation quality of morels to facilitate subsequent seeding and yield improvement. In the disclosure, the mushroom strain bag is cut off and flipped on the ground directly, so that mycelia in the mushroom strain bag grow up in a circular manner and morels fruit in a circular and diffuse manner
- 2. As required by the disclosure, the soil layer covering on the composts is 1-2 cm thick; with this requirement of the thickness, the soil layer can achieve the objective of separating microbes, while allowing mycelia to penetrate the soil layer; if the soil layer is too thick or too thin, the yield stability and yield of morels will be influenced.
- 3. The mushroom strain bag of the disclosure includes a body and a cap, the cap is capped on the body, air holes are arranged at the side or bottom of the body, and a layer of air-permeable filter material is covered on the air holes. The composts and the soil layer are needed to fill the entire mushroom strain bag. Such structure of the mushroom strain bag can play roles in improving the compactness of the mushroom strain bag and separating microbes; air holes have air permeability.
- 4. As required by the disclosure, the surface soil moisture is 70-85%; this soil moisture contributes to the production of morels and the improvement of the yield of morels.

### BRIEF DESCRIPTION OF THE DRAWINGS

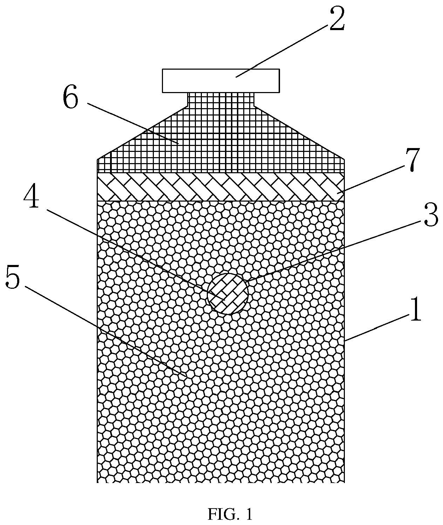

FIG. 1 schematically illustrates the structure of a mushroom strain bag of the disclosure.

In the drawing: 1 denotes a body, 2 denotes a cap, 3 denotes air holes, 4 denotes a layer of air-permeable filter material, 5 denotes composts, 6 denotes a soil layer, and 7 denotes mycelia.

### DETAILED DESCRIPTION

The disclosure provides a planting method for morels, including the following steps:
- step 1, preparing spawns
- 1.1 filling composts (5) and the soil layer an entire mushroom strain bag wherein the composts are compressed and the soil layer is 1-2 cm thick, covering a layer of loose soil (6) on the composts, sealing the mushroom strain bag for sterilization and sterilizing the mushrooms strain bag;
- 1.2 after sterilization, opening the mushroom strain bag for inoculation, inoculating the mushroom strain bag, the mushroom strain bag is sealed and going to the next step until morel mycelia (7) grow down to at least 2-3 cm of the soil layer;
- step 2, flipping the spawns cutting off the mushroom strain bag, flipping on the ground directly, and compressing, where the spacing between mushroom strain bags is 30-35 cm, surface soil is 16-25 cm thick, a soil tank is 100-120 cm wide, and mycelia grow up in a circular manner around the mushroom strain bag;
- step 3, managing the humidity surface soil moisture is 70-85%;
- step 4, fruiting for harvesting.
fruiting for harvesting 70-100 days after the mushroom strain bag is flipped on the ground.

In step 1.1, the composts are available from either those in the existing cultivation of morels, or those prepared by the following method: wheats are soaked in saturated limewater for one day until the wheat expansion rate is no greater than 50%, and then well-soaked corncobs and other components are mixed therein to prepare the composts.

In step 1.2, when inoculating, it is determined whether the mushroom strain bag is inoculated in an aseptic environment according to the compactness thereof; if compact, the bag can be inoculated in the presence of microbes; if loose, the bag should be inoculated in an aseptic environment; after inoculation, the mushroom strain bag is capped and sealed.

The disclosure will be further described below in conjunction with examples, and the described examples are only a part of, not all of, the examples of the disclosure.

### Example 1

A planting method for morels was provided, including the following steps:
- step 1, preparing spawns
   ∘ 1.1 filling composts in a mushroom strain bag, covering a layer of loose soil on the composts, and sealing the mushroom strain bag for sterilization, where the soil layer was 1 cm thick, the mushroom strain bag included a body and a cap, the cap was capped on the body, air holes were arranged at the side or bottom of the body, a layer of air-permeable filter material was covered on the air holes, and the composts and the soil layer were needed to fill the entire mushroom strain bag;
   ∘ 1.2 after sterilization, opening the mushroom strain bag for inoculation, and going to the next step until morel mycelia grew down to at least 2 cm of the soil layer;
- step 2, flipping the spawns cutting off the mushroom strain bag, flipping on the ground directly, and compressing, where the spacing between mushroom strain bags was 30 cm, surface soil was 16 cm thick, a soil tank was 120 cm wide, and mycelia grew up in a circular manner around the mushroom strain bag;
- step 3, managing the humidity surface soil moisture was 70%;
- step 4, fruiting for harvesting.
fruiting for harvesting 75 days after the mushroom strain bag was flipped on the ground.

### Example 2

A planting method for morels was provided, including the following steps:
- step 1, preparing spawns
   ∘ 1.1 filling composts in a mushroom strain bag, covering a layer of loose soil on the composts, and sealing the mushroom strain bag for sterilization, where the soil layer was 1.5 cm thick, the mushroom strain bag included a body and a cap, the cap was capped on the body, air holes were arranged at the side or bottom of the body, a layer of air-permeable filter material was covered on the air holes, and the composts and the soil layer were needed to fill the entire mushroom strain bag;
   ∘ 1.2 after sterilization, opening the mushroom strain bag for inoculation, and going to the next step until morel mycelia grew down to at least 2.5 cm of the soil layer;
- step 2, flipping the spawns cutting off the mushroom strain bag, flipping on the ground directly, and compressing;
- step 3, managing the humidity surface soil moisture was 80%;
- step 4, fruiting for harvesting.
fruiting for harvesting 70 days after the mushroom strain bag was flipped on the ground.

### Example 3

A planting method for morels was provided, including the following steps:
- step 1, preparing spawns
   ∘ 1.1 filling composts in a mushroom strain bag, covering a layer of loose soil on the composts, and sealing the mushroom strain bag for sterilization, where the soil layer was 1.8 cm thick, the mushroom strain bag included a body and a cap, the cap was capped on the body, air holes were arranged at the side or bottom of the body, a layer of air-permeable filter material was covered on the air holes, and the composts and the soil layer were needed to fill the entire mushroom strain bag;
   ∘ 1.2 after sterilization, opening the mushroom strain bag for inoculation, and going to the next step until morel mycelia grew down to at least 2.8 cm of the soil layer;
- step 2, flipping the spawns cutting off the mushroom strain bag, flipping on the ground directly, and compressing, where the spacing between mushroom strain bags was 32 cm, surface soil was 20 cm thick, a soil tank was 110 cm wide, and mycelia grew up in a circular manner around the mushroom strain bag;
- step 3, managing the humidity surface soil moisture was 75%;
- step 4, fruiting for harvesting.
fruiting for harvesting 87 days after the mushroom strain bag was flipped on the ground.

### Example 4

A planting method for morels was provided, including the following steps:
- step 1, preparing spawns
   ∘ 1.1 filling composts in a mushroom strain bag, covering a layer of loose soil on the composts, and sealing the mushroom strain bag for sterilization, where the soil layer was 2 cm thick, the mushroom strain bag included a body and a cap, the cap was capped on the body, air holes were arranged at the side or bottom of the body, a layer of air-permeable filter material was covered on the air holes, and the composts and the soil layer were needed to fill the entire mushroom strain bag;
   ∘ 1.2 after sterilization, opening the mushroom strain bag for inoculation, and going to the next step until morel mycelia grew down to at least 3 cm of the soil layer;
- step 2, flipping the spawns cutting off the mushroom strain bag, flipping on the ground directly, and compressing, where the spacing between mushroom strain bags was 35 cm, surface soil was 18 cm thick, a soil tank was 100 cm wide, and mycelia grew up in a circular manner around the mushroom strain bag;
- step 3, managing the humidity surface soil moisture was 85%;
- step 4, fruiting for harvesting.
fruiting for harvesting 93 days after the mushroom strain bag was flipped on the ground.

### Example 5

A planting method for morels was provided, including the following steps:
- step 1, preparing spawns
   ∘ 1.1 filling composts in a mushroom strain bag, covering a layer of loose soil on the composts, and sealing the mushroom strain bag for sterilization, where the soil layer was 2 cm thick, the mushroom strain bag included a body and a cap, the cap was capped on the body, air holes were arranged at the side or bottom of the body, a layer of air-permeable filter material was covered on the air holes, and the composts and the soil layer were needed to fill the entire mushroom strain bag;
   ∘ 1.2 after sterilization, opening the mushroom strain bag for inoculation, and going to the next step until morel mycelia grew down to at least 2 cm of the soil layer;
- step 2, flipping the spawns cutting off the mushroom strain bag, flipping on the ground directly, and compressing, where the spacing between mushroom strain bags was 35 cm, surface soil was 16 cm thick, a soil tank was 118 cm wide, and mycelia grew up in a circular manner around the mushroom strain bag;
- step 3, managing the humidity surface soil moisture was 82%;
- step 4, fruiting for harvesting.
fruiting for harvesting 100 days after the mushroom strain bag was flipped on the ground. Comparison of yields in all examples is shown in Table 1.

| Example | Planting area (mu) | Yield (catty) |
|---|---|---|
| Example 1 | 1 | 905 |
| Example 2 | 1 | 860 |
| Example 3 | 1 | 950 |
| Example 4 | 1 | 875 |
| Example 5 | 1.2 | 1,030 |

## Claims

1. A planting method for morels, comprising the following steps:
step 1, preparing spawns, comprising
1.1 filling composts (5) and the soil layer an entire mushroom strain bag wherein the composts are compressed and the soil layer is 1-2 cm thick, covering a layer of loose soil (6) on the composts, sealing the mushroom strain bag for sterilization and sterilizing the mushrooms strain bag;
1.2 after sterilization, opening the mushroom strain bag for inoculation, inoculating the mushroom strain bag, the mushroom strain bag is sealed and going to the next step until morel mycelia (7) grow down to at least 2-3 cm of the soil layer;
step 2, flipping the spawns, comprising:
2.1 cutting off the mushroom strain bag, flipping on the ground directly, and compressing;
step 3, managing the humidity, wherein surface soil moisture is required at 70-85%;
step 4, fruiting for harvesting.

2. The planting method for morels according to claim 1, wherein in step 1.1, the mushroom strain bag comprises a body (1) and a cap (2), the cap is capped on the body, air holes (3) are arranged at the side or bottom of the body, and a layer of air-permeable filter material (4) is covered on the air holes.

3. The planting method for morels according to claim 1, wherein in step 2, the spacing between mushroom strain bags is 30-35 cm, surface soil is 16-25 cm thick, and a soil tank is 100-120 cm wide.

4. The planting method for morels according to claim 1, wherein in step 3, said fruiting for harvesting is for 70-100 days after the mushroom strain bag is flipped on the ground.

## Patentansprüche

1. Ein Pflanzverfahren für Morcheln, das die folgenden Schritte beinhaltet:
Schritt 1, Vorbereiten von Myzelien, das Folgendes beinhaltet:
1.1 Füllen eines ganzen Pilzstammbeutels mit Komposten (5) und der Erdschicht, wobei die Komposte komprimiert werden und die Erdschicht 1-2 cm dick ist, Abdecken der Komposte mit einer Schicht loser Erde (6), Verschließen des Pilzstammbeutels zur Sterilisation und Sterilisieren des Pilzstammbeutels;
1.2 nach der Sterilisation, Öffnen des Pilzstammbeutels zur Inokulation, Inokulieren des Pilzstammbeutels, der Pilzstammbeutel wird verschlossen und Übergehen zum nächsten Schritt, sobald die Morchelmyzelien (7) auf mindestens 2-3 cm unter die Bodenschicht gewachsen sind;
Schritt 2, Kippen der Myzelien, das Folgendes beinhaltet:
2.1 Aufschneiden des Pilzstammbeutels, direktes Kippen auf den Boden und Zusammendrücken;
Schritt 3, Regeln der Feuchtigkeit, wobei eine Erdfeuchtigkeit an der Oberfläche von 70-85 % erforderlich ist;
Schritt 4, Fruchtbildung für die Ernte.

2. Pflanzverfahren für Morcheln gemäß Anspruch 1, wobei in Schritt 1.1 der Pilzstammbeutel einen Körper (1) und eine Haube (2) beinhaltet, die Haube auf den Körper aufgesetzt ist, Luftlöcher (3) an der Seite oder an dem Boden des Körpers angeordnet sind und eine Schicht aus luftdurchlässigem Filtermaterial (4) die Luftlöcher bedeckt.

3. Pflanzverfahren für Morcheln gemäß Anspruch 1, wobei in Schritt 2 der Abstand zwischen den Pilzstammbeuteln 30-35 cm beträgt, die Oberflächenerde 16-25 cm dick ist und ein Erdbehälter 100-120 cm breit ist.

4. Pflanzverfahren für Morcheln gemäß Anspruch 1, wobei in Schritt 3 die Fruchtbildung für die Ernte 70-100 Tage dauert, nachdem der Pilzstammbeutel auf den Boden gekippt wurde.

## Revendications

1. Un procédé de plantation pour morilles, comprenant les étapes suivantes :
étape 1, préparer des blancs, ce qui comprend
1.1 remplir de composts (5) et de la couche de terre un sac entier de souches de champignons où les composts sont comprimés et la couche de terre fait 1 à 2 cm d'épaisseur, recouvrir d'une couche de terre meuble (6) les composts, sceller le sac de souches de champignons pour stérilisation et stériliser le sac de souches de champignons ;
1.2 après stérilisation, ouvrir le sac de souches de champignons pour ensemencement, ensemencer le sac de souches de champignons, le sac de souches de champignons est scellé et passer à l'étape suivante dès que les mycéliums de morilles (7) ont poussé vers le bas jusqu'à au moins 2 à 3 cm de la couche de terre ;
étape 2, renverser les blancs, ce qui comprend :
2.1 découper le sac de souches de champignons, renverser directement sur le sol, et comprimer ;
étape 3, réguler l'humidité, une teneur en humidité de terre en surface de l'ordre de 70 à 85 % étant requise ;
étape 4, faire fructifier pour la récolte.

2. Le procédé de plantation pour morilles selon la revendication 1, où à l'étape 1.1, le sac de souches de champignons comprend un corps (1) et un bouchon (2), le bouchon bouche le corps, des trous d'aération (3) sont agencés sur le côté ou le fond du corps, et une couche de matière filtrante perméable à l'air (4) recouvre les trous d'aération.

3. Le procédé de plantation pour morilles selon la revendication 1, où à l'étape 2, l'espacement entre sacs de souches de champignons fait 30 à 35 cm, la terre en surface fait 16 à 25 cm d'épaisseur, et un bac de terre fait 100 à 120 cm de large.

4. Le procédé de plantation pour morilles selon la revendication 1, où à l'étape 3, ledit fait de faire fructifier pour la récolte dure 70 à 100 jours après que le sac de souches de champignons a été renversé sur le sol.
